# EUROPEAN PATENT SPECIFICATION

(11) **EP 2 680 811 B1**
(45) Date of publication and mention of the grant of the patent: **11.04.2018**
(21) Application number: 12707077.9
(22) Date of filing: 28.02.2012
(51) Int. Cl.: A61K 8/60, A61Q 17/04, A61Q 19/04, A61K 8/04, A61Q 19/00, A61Q 19/08

(54) **NOVEL USE OF STEVIOL**
NEUE VERWENDUNG VON STEVIOL
NOUVELLE UTILISATION DE STÉVIOL

(30) Priority: 01.03.2011 EP 11156477; 03.03.2011 EP 11156864; 19.04.2011 EP 11162951
(43) Date of publication of application: 08.01.2014
(73) Proprietor: DSM IP Assets B.V., 6411 TE Heerlen (NL)
(72) Inventor: GORALCZYK, Regina, CH-4002 Basel (CH); GRÄUB, Remo, CH-4002 Basel (CH); IMFELD, Dominik, CH-4002 Basel (CH); VOLLHARDT, Jürgen H., CH-4002 Basel (CH)
(74) Representative: Berg, Katja
(86) International application number: PCT/EP2012/053372
(87) International publication number: WO 2012/116990

(56) References cited:
- WO-A1-2011/068147
- WO-A1-2013/093880
- WO-A2-2012/010624
- WO-A2-2012/116992
- US-A1- 2012 021 088
- "Priority application of WO 2012/010624 A2", File inspection of WO 2012/010624 A2 , 14 February 2012 (2012-02-14), XP002720448, Retrieved from the Internet: URL:https://register.epo.org/application?n umber=EP11735642&lng=en&tab=doclist
- "Research disclosure", RESEARCH DISCLOSURE, MASON PUBLICATIONS, HAMPSHIRE, GB, vol. 565, no. 63, 1 May 2011 (2011-05-01), page 619, XP007140554, ISSN: 0374-4353
- S. MADAN ET AL.: "Stevia rebaudiana (Bert.) Bertoni - A Review", INDIAN JOURNAL OF NATURAL PRODUCTS AND RESOURCES, vol. 1, no. 3, September 2010 (2010-09), pages 267-286, XP002720000,
- "JSID Abstracts", JOURNAL OF DERMATOLOGICAL SCIENCE, ELSEVIER SCIENCE PUBLISHERS, SHANNON, IE, vol. 56, no. 3, 1 December 2009 (2009-12-01), pages E1-E49, XP026789084, ISSN: 0923-1811 [retrieved on 2009-11-07]

## Description

The present invention relates to a cosmetic composition as defined in claim 1. Today, it is important to look healthy and a tanned skin is always a sign of good health. One method of obtaining a tan is to expose skin to UV radiation causing direct DNA damage to the skin, which the body naturally combats and seeks to repair. In the process of repairing the damage and protect the skin, the body creates and releases the brown-colored pigment called melanin into the skin's cells, which gives the skin a darker tone. Melanin is produced by cells called melanocytes and protects the body from direct and indirect DNA damage by absorbing an excess of solar radiation. However, as exposure to UV radiation may have detrimental health effects such as sunburn or even skin cancer many people prefer to use chemical products which can produce a tanning result without exposure to ultraviolet radiation.

Today, most of the cosmetic products intended for artificially tanning of the skin comprise carbonyl compounds such as dihydroxyacetone (DHA) or erythrulose which cause a chemical reaction with the amino acids in the dead layer on the skin surface thus forming colored species. However, the drawback of these compounds is that the tan produced therewith, in contrast to sun tanned skin, does not protect against UV-radiation and the achieved skin color does not correspond exactly to naturally sun tanned skin and the tan often is uneven.
Thus, there is an ongoing need for compounds which stimulate the production of melanin in skin melanocytes similarly to the production of melanin stimulated by DNA damage induced by UVB-radiation, which in turn leads to the development of a natural skin tan.

Surprisingly it has been found the use of steviol overcomes the drawbacks of the prior art and activates the melanin formation in human skin melanocytes and can thus be used for sunless tanning. The formulations containing steviol also have the advantage of being slightly colored on application to the skin and thus of being able to be dosed and to visualize the zone of application of the product. Furthermore, the change in pigmentation (i.e. increased melanin content) will render the skin better-prepared to deal with direct sunlight, thereby reducing the risk and/or severity of skin problems such as sunburns, premature wrinkling and aging, skin cancer, etc. In particular need for such more protection without UV radiation are fairly tanned individuals or immunosuppressed patients.

Thus, the invention relates to a cosmetic composition as defined in claim 1. Due to the increased skin tan (skin pigmentation) the skin of humans which topically apply steviol is better protected against skin damages due to ultraviolet radiation. Steviol may be used as such or in the form of a cosmetically acceptable salt formed from steviol and a suitable base. Suitable salts encompass all salts with a cosmetically acceptable cation. The term cosmetically acceptable cation refers to any organic or metal cation that is not toxic to the skin and/or does not cause allergic reactions. Examples of suitable salts encompass e.g. the respective ammonium, alkyl ammonium or mono-, di- and triethanolammonium salts as well as the alkaline or alkaline earth metal salts such as the sodium, potassium, calcium or magnesium salts. Particularly suitable salts are the respective triethanolammonium salts as well as the sodium or potassium salts. Steviol may be used in free or in encapsulated form, for example in lipid vesicles such as liposomes such as e.g. disclosed in WO97/25970, in nanosomes or in cyclodextrins.

The cosmetic composition for use as defined in claim 1 is especially attractive, since many people have a special interest in cosmetic treatments considered as "natural" with mild effects and without major side effects. Steviol is preferably used in the form of topical compositions. The topical compositions advantageously comprise from 0.0001-20 wt.-% of steviol based on the total weight of the composition. Advantageously, the topical compositions comprise from 0.01-5 wt.-%, such as particularly from 0.01-1 wt.-%, of steviol based on the total weight of the composition.

Due to the increased skin tan (skin pigmentation) the skin of humans which topically apply steviol is better protected against skin damages due to ultraviolet radiation.

The term "effective amount" means an amount necessary to obtain a desired physiological effect.

The physiological effect may be achieved by one single dose or by repeated doses. The dosage administered may, of course, vary depending upon known factors, such as the physiological characteristics of the particular composition; the age, health and weight of the recipient; the nature and extent of the symptoms; the kind of concurrent treatment; the frequency of treatment; and/or the effect desired and can be adjusted by a person skilled in the art. Preferably, 1 g cream per cm² skin is applied once a day.

The term "topical composition" as used herein denotes to any composition suitable for the topical application to mammalian keratinous tissue such as in particular to human skin. In particular, the topical compositions according to the present invention are cosmetic compositions that can be topically applied to mammalian keratinous tissue, particularly to human skin. Thus, the topical compositions according to the present invention are in particular cosmetic skin care compositions comprising steviol and a cosmetically acceptable carrier.
The term "cosmetic preparation" or "cosmetic composition" as used in the present application refers to cosmetic compositions as defined under the heading "Kosmetika" in Römpp Lexikon Chemie, 10th edition 1997, Georg Thieme Verlag Stuttgart, New York as well as to cosmetic compositions as disclosed in A. Domsch, "Cosmetic Preparations", Verlag für chemische Industrie (ed. H. Ziolkowsky), 4th edition, 1992.

The term cosmetically acceptable carrier refers to all carriers and/or excipients and/ or diluents conventionally used in cosmetic compositions.
It is advantageous if the topical compositions according to the invention exhibit a pH of 8 or less such as in particular a pH in the range of 2.5 to 8, more in particular in the range of 3 to 7.5 and particularly in the range of 4 to 7 as Steviol, shows a significant lower degree of discoloration at lower pH as illustrated in the examples. The pH of the topical composition can be adjusted with conventional acids, bases or buffering solutions according to methods well known to a person skilled in the art.
According to a further advantageous embodiment, the topical compositions according to the present invention in addition contain at least one stabilizer and/or at least one photoprotective agent and/or at least one wetting agent and/or at least one penetrant.

In order to improve the stability of steviol the compositions according to the invention advantageously include one or more stabilizers such as e.g. antioxidants or chelating agents.

Suitable antioxidants/ chelating agents to be incorporated into the topical compositions according to the present invention are basically all known antioxidants/ chelating agents usually formulated into topical and in particular cosmetic compositions. Especially preferred are antioxidants/ chelating agents chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol to µmol/ kg), additionally (metal)-chelators (such as α-hydroxyfatty acids (citric acid, lactic acid, malic acid), palmic-, phytinic acid, lactoferrin), β-hydroxyacids, huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, Tetradibutyl Pentaerithrityl Hydroxyhydrocinnamate (Tinogard TT), Tetrabutyl Ethylidinebisphenol (Tinogard NOA), Octadecyl Di-t-butyl-4-hydroxyhydrocinnamate (Tinogard TS) trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients, or enzymes such as superoxide dismutase, catalase or similar, or activators of such enzymes. The one or more antioxidant/ chelating agent may be present in an amount of at least 0.01 wt.- % such as in an amount of 0.01 to about 10 wt.- % and particularly in an amount of 0.1 to about 1 wt.-% based on the total weight of the composition.

Particularly suited antioxidants for the use in the topical compositions according to the invention encompass vitamin E and its derivatives such as particularly tocopheryl acetate. Tocopheryl acetate may be present in the topical compositions in an amount from about 0.05 wt.-% to 25 wt.-%, in particular 0.05 wt.-% to 5 wt.-%. Another vitamin E derivative of interest is tocopheryl linoleate. Tocopheryl linoleate may be present in the topical composition in an amount from about 0.05 wt.-% to 25 wt.-% in particular 0.05 wt.-% to 5 wt.-%.

Another suitable antioxidant is vitamin A and/or its derivatives. In particular retinoid derivatives such as retinyl palmitate or retinyl propionate is used in the topical compositions according to the invention in an amount of 0.01 to 5 wt.-%, in particular 0.01 to 0.3 wt.-%. The vitamin A and/ or its derivatives can also be used in an encapsulated form.

Another particular suitable antioxidant is Vitamin C (ascorbic acid) and/or its derivatives. In particular ascorbyl phosphate such as Stay C (sodium ascorbyl monophosphate) Mg ascorbylphosphate and/ or Ascorbylglucoside is used in the topical compositions according to the invention in an amount of 0.1 to 5 wt.-% in particular of 0.1 to 2 wt.-%.

Suitable photoprotective agents which may be incorporated into the topical compositons according to the present invention include conventional UV-filter substances. The UV-filter substances are advantageously selected from among acrylates such as 2-ethylhexyl 2-cyano-3,3-diphenylacrylate (octocrylene, PARSOL® 340), ethyl 2-cyano-3,3-diphenylacrylate and the like; camphor derivatives such as 4-methyl benzylidene camphor (PARSOL® 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like; cinnamate derivatives such as ethylhexyl methoxycinnamate (PARSOL® MCX), ethoxyethyl methoxycinnamate, diethanolamine methoxycinnamate (PARSOL® Hydro), isoamyl methoxycinnamate and the like as well as cinnamic acid derivatives bond to siloxanes; p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate; benzophenones such as benzophenone-3, benzophenone-4, 2,2',4,4'-tetrahydroxy-benzophenone, 2,2'-dihydroxy-4,4'-dimethoxybenzophenone and the like; esters of benzalmalonic acid such as di-(2-ethylhexyl) 4-methoxybenzalmalonate; esters of 2-(4-ethoxy-anilinomethylene)propandioic acid such as 2-(4-ethoxy anilinomethylene) propandioic acid diethyl ester as described in the European Patent Publication EP 0895 776; organosiloxane compounds containing benzmalonate groups as described in the European Patent Publications EP 0358584 B1, EP 0538431 B1 and EP 0709080 A1 such as polysilicone-15 (PARSOL® SLX); drometrizole trisiloxane (Mexoryl® XL); imidazole derivatives such as e.g. 2-phenyl benzimidazole sulfonic acid and its salts (PARSOL® HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like; salicylate derivatives such as isopropylbenzyl salicylate, benzyl salicylate, butyl salicylate, ethylhexyl salicylate (PARSOL® EHS, Neo Heliopan® OS), isooctyl salicylate or homomenthyl salicylate (homosalate, PARSOL® HMS, Neo Heliopan® HMS) and the like; triazine derivatives such as ethylhexyl triazone (Uvinul® T-150), diethylhexyl butamido triazone (Uvasorb® HEB), 2,4,6-Tris-(biphenyl)1,3,5-triazine and the like, merocyanines as e.g. disclosed in DE10 2007 024 345 on page 4, paragraph 19 which are incorporated by reference herein, encapsulated UV-filters such as encapsulated ethylhexyl methoxycinnamate (Eusolex® UV-pearls) or microcapsules loaded with UV-filters as e.g. disclosed in EP 1471995 and the like; dibenzoylmethane derivatives such as 4-tert.-butyl-4'-methoxydibenzoyl-methane (PARSOL® 1789) or isopropyldibenzoylmethane and the like; benzotriazole derivatives such as 2,2'-methylene-bis-(6-(2H-benzotriazole-2-yl)-4-(1,1,3,3,-tetramethylbutyl)-phenol (Tinosorb® M) and the like; bis-ethylhexyloxyphenol methoxyphenyl triazine (Tinosorb® S) and the like; phenylene-1,4-bis-benzimidazolsulfonic acids or salts such as 2,2-(1,4-phenylene)bis-(1H-benzimidazol-4,6-disulfonic acid) (Neo Heliopan® AP); amino substituted hydroxybenzophenones such as 2-(4-Diethylamino-2-hydroxy-benzoyl)-benzoic acid hexylester (Uvinul® A plus) or 1,1'-(1,4-piperazinediyl)bis[1-[2-[4-(diethylamino)-2-hydroxybenzoyl]phenyl]-methanone (CAS No 919803-06-8); Ionic UV-A filters as described in the International Patent Publication WO2005080341 A1; pigments such as microparticulated ZnO or TiO₂ and the like. The term "microparticulated" refers to a particle size from about 5 nm to about 200 nm, particularly from about 15 nm to about 100 nm. The pigments may also be coated by other metal oxides such as e.g. aluminum or zirconium oxides or by organic coatings such as e.g. polyols, methicone, aluminum stearate, alkyl silane. Such coatings are well known in the art. Furthermore, the pigments (ZnO, TiO₂) can be used in the form of commercially available oily or aqueous pre-dispersions. These pre-dispersions may further contain a dispersing aid and/ or solubilisator.

Particularly preferred UV-filter substances are the commercially available and widely used UV-filter substances octocrylene (PARSOL® 340), 4-methyl benzylidene camphor (PARSOL® 5000), ethylhexyl methoxycinnamate (PARSOL® MCX), ethylhexyl triazone (Uvinul® T-150), diethylhexyl butamido triazone (Uvasorb® HEB), 2,2'-methylene-bis-(6-(2H-benzotriazole-2-yl)-4-(1,1,3,3,-tetramethylbutyl)-phenol (Tinosorb® M), bis-ethylhexyloxyphenol methoxyphenyl triazine (Tinosorb® S), 2,2-(1,4-phenylene)bis-(1H-benzimidazol-4,6-disulfonic acid (NeoHeliopan® AP), 2-(4-Diethylamino-2-hydroxybenzoyl)-benzoic acid hexylester (Uvinul® A plus), 1,1'-(1,4-piperazinediyl)bis[1-[2-[4-(diethylamino)-2-hydroxybenzoyl]phenyl]-methanone (CAS No 919803-06-8), polysilicone-15 (PARSOL® SLX), 2-phenyl benzimidazole sulfonic acid (PARSOL® HS), ethylhexyl salicylate (PARSOL® EHS), homomenthyl salicylate (PARSOL® HMS), Benzophenone-3 (Uvinul® M 40), Benzophenone-4 (Uvinul® MS 40), Butyl Methoxydibenzoyl Methane (Parsol® 1789), Terephtalidene dicampher Sulfonic Acid (Mexoryl® SX), Drometrizole Trisiloxane (Mexoryl® XL), microfine zinc or titanium dioxide such as in particular PARSOL® TX as well as mixtures thereof.
The photoprotective agents (in total) are generally present in the topical compositions according to the invention comprising steviol in proportions ranging from 0.1 to 30 wt.-%, preferably ranging from 0.2 to 15wt.-%, most preferably ranging from 0.5 to 10wt.-% based on the total weight of the composition.
In order to increase the remanence of the skin color and/or the homogeneity of the color, the compositions according to the invention comprising steviol may additionally comprise at least a wetting agent and/or penetrant such as for instance urea, hydroxyethylurea, polyols such as glycerol, alkylene glycols such as propylene glycol or butylene glycol, or alkylene glycol alkyl ethers such as propylene glycol monomethyl ether.
In order to adjust the color obtained via the tanning process according to the invention and to better adapt it to the various types of skin tone, the topical compositions comprising steviol also comprise one or more coloring agents selected from erythrulose and/or dihydroxyacetone. Additional coloring agents may be present and they may be selected especially from natural and synthetic direct dyes. They may be organic or mineral dyes. The mineral dyes may be, for example, iron oxide pigments whose mean elementary particle size is less than 100 nm, such as those described in EP-966,953. The natural or synthetic liposoluble organic dyes are, for example, DC Red 17, DC Red 21, DC Red 27, DC Green 6, DC Yellow 11, DC Violet 2, DC Orange 5, Sudan red, carotenes ([beta]-carotene or lycopene), xanthophylls (capsanthin, capsorubin or lutein), palm oil, Sudan brown, quinoline yellow, annatto and curcumin.
The natural or synthetic water-soluble dyes are, for example, FDC Red 4, DC Red 6, DC Red 22, DC Red 28, DC Red 30, DC Red 33, DC Orange 4, DC Yellow 5, DC Yellow 6, DC Yellow 8, FDC Green 3, DC Green 5, FDC Blue 1, betanin (beetroot), carmine, copper-containing chlorophylline, methylene blue, anthocyanins (enocyanin, black carrot, hibiscus or elder) and riboflavin.
The dyes may also be selected from among anthraquinones, caramel, carmine, carbon black, azulene blues, methoxalene, trioxalene, guajazulene, chamuzulene, rose Bengal, cosine 10B, cyanosin, daphinine, juglone, lawsone, extracts of fermented soya, of algae, of fungi or of microorganisms, flavylium salts not substituted in position 3, for instance those described in EP-1-172,091, extracts of Gesneria fulgens, Blechum procerum or Saxifraga and pigments that may be obtained by extraction with an organic or aqueous-organic solvent of a culture medium of micromycetes of the Monascus type.
These dyes may also be selected from among indole derivatives, for instance the monohydroxyindoles as described in FR-2,651,126 (i.e.: 4-, 5-, 6- or 7-hydroxyindole) or the dihydroxyindoles as described in EP-B-0-425,324 (i.e.: 5,6-dihydroxyindole, 2-methyl-5,6-dihydroxyindole, 3-methyl-5,6-dihydroxyindole or 2,3-dimethyl-5,6-dihydroxyindole).

Further coloring agents are e.g. self-tanning agents such as melanin derivates which may be incorporated into the topical compositions according to the invention. Advantageously the topical compositions according to the present invention contain dihydroxyacetone (DHA) and/ or erythrulose in an amount of 1 to 10 wt.-% based on the total weight of the composition according to the invention.
The topical compositions according to the invention may further comprise biological actives selected from general activators of melanogenesis like tyrosinase activators, peptide hormones, MCR-1 agonists, MITF stimulators, cAMP stimulators (forskulin), cAMP-activators (caffeine) and neurotrophins.
Preferred tyrosinase activators are any substance which increases tyrosinase expression or enzyme activity, like e.g. glycyrrhizin from the root of licorice or glycyrrhetic acid.

The topical compositions according to the invention preferably contain at least one tyrosinase activator in an amount (in total) of 0.01 to 1 wt.-%, preferably 0.1 to 0.5 wt.-% based on the total weight of the composition.
Peptide hormones belonging to the group of melanocortins are the preferred peptide hormones including ACTH, alpha-MSH, beta-MSH and gamma-MSH or their synthetically modified analogues Melanotan I and II; these peptides are all cleavage products of a large precursor peptide called pro-opiomelanocortin (POMC). Alpha-MSH is the most important melanocortin for pigmentation. The melanocyte-stimulating hormones (collectively referred to as MSH or intermedins) are a class of peptide hormones that in nature are produced by cells in the intermediate lobe of the pituitary gland. They stimulate the production and release of melanin (melanogenesis) by melanocytes in skin. Therefore, they will be advantageously combined with steviol. Of particular interest is the combination of steviol according to this invention with tri or tetra-peptides representing the recognition sequence in the synthetically modified analogues Melanotan I and II, which is His-D-Phe-Arg-Trp. These tri- or the tetra-peptides may have a hydrophobic modification on the C- or N-terminus for better skin penetration.

A list of further peptides, hydrophobically modified or not for combination with steviol can be found in US2008200396.

The topical compositions according to the invention comprising steviol may also comprise additional active agents selected especially from moisturizers, further skin tanning agents, desquamating agents, agents for improving the barrier function, dermo-decontracting agents, anti-glycation agents, agents for stimulating the synthesis of dermal and/or epidermal macromolecules and/or for preventing their degradation, agents for stimulating fibroblast or keratinocyte proliferation and/or keratinocyte differentiation, agents for promoting the maturation of the horny envelope, NO-synthase inhibitors, peripheral benzodiazepine receptor (PBR) antagonists, agents for increasing the activity of the sebaceous glands, agents for stimulating the energy metabolism of cells, tensioning agents, lipo-restructuring agents, slimming agents, agents for promoting the cutaneous capillary circulation, calmatives and/or anti-irritants, sebo-regulators or anti-seborrhoeic agents, astringents, cicatrizing agents, anti-inflammatory agents and anti-acne agents.
One skilled in the art will select the said active agent(s) as a function of the effect desired on the skin.
Particularly suitable moisturizers for the incorporation into the topical compositions according to the invention are glycerine, lactic acid and/ or lactates, in particular sodium lactate, butylene glycol, propylene glycol, biosaccaride gum-1, glycine soja, ethylhexyloxyglycerin, pyrrolidoncarboxy acid, hydroxyethylurea and urea. It is further advantageous to use polymeric moisturizer such as water soluble or water gelifiable polysaccharides. In particular advantageous are e.g. hyaluronic acid, chitosan, Pentavitin and/ or a polysaccharid rich in fucose [CAS No 178463-23-5, commercially available as Fucogel®1000 by SOLABIA S.A.]. The moisturizers can also be used as anti-ageing ingredients such as e.g. for the treatment of photo-aged skin.
The topical compositions according to the invention preferably contain at least one moisturizer in an amount (in total) of 0.1 to 20 wt.-%, preferably 0.5 to 10 wt.-% based on the total weight of the composition.

Further examples of cosmetically active ingredients suitable to be used in the topical composition according to the invention comprising steviol comprise peptides (e.g., Matrixyl™ [pentapeptide derivative]), oligopeptides, wax-based synthetic peptides (e.g., octyl palmitate and tribehenin and sorbitan isostearate and palmitoyl-oligopeptide), glycerol, alpha-glycosylrutin, natural or synthetic flavanoids or isoflavanoids, creatine, creatinine, guanidine (e.g. amino guanidine); vitamins and derivatives thereof such as vitamin C (ascorbic acid), vitamin A (e.g., retinoid derivatives such as retinyl palmitate or retinyl propionate), vitamin E (e.g., tocopherol acetate), vitamin B₃ (e.g. niacinamide) and vitamin B₅ (e.g. panthenol), vitamin B₆ and vitamin B₁₂, biotin, folic acid; anti-acne actives or medicaments (e.g. resorcinol, salicylic acid, and the like); antioxidants (e.g. phytosterols, lipoic acid); flavonoids (e.g. isoflavones, phytoestrogens); skin soothing and healing agents such as aloe vera extract, allantoin and the like; agents suitable for aesthetic purposes such as essential oils, fragrances, skin sensates, opacifiers, aromatic compounds (e.g., clove oil, menthol, camphor, eucalyptus oil, and eugenol), desquamatory actives, hydroxy acids such as AHA acids, poly unsaturated fatty acids, radical scavengers, farnesol, antifungal actives in particular bisabolol, alkyldiols such as 1,2-pentanediol, hexanediol or 1,2-octanediol, phytol, polyols such as phytanetriol, ceramides and pseudoceramides, amino acids, protein hydrolysates, polyunsaturated fatty acids, plant extracts like kinetin, DNA or RNA and their fragmentation products, carbohydrates, conjugated fatty acids, carnitin, carnosine, biochinonen, phytofluen, phytoen, and their corresponding derivatives and co-enzyme Q10 (ubiquinone) without being limited thereto.

The additional cosmetically active ingredient is typically included in an amount of at least 0.001 wt.-% based on the total weight of the topical composition. Generally, an amount of about 0.001 wt.-% to about 30 wt.-%, preferably from about 0.001 wt.-% to about 10 wt.-% of an additional cosmetically active agent is used.

Particularly preferred examples of ingredients to be used in the compositions according to the invention are vitamin C (ascorbic acid) and/or its derivatives (e.g. ascorbyl phosphate such as Stay C (sodium ascorbyl monophosphate) from DSM Nutritional Products Ltd.), vitamin A and/or its derivatives (e.g., retinoid derivatives such as retinyl palmitate or retinyl propionate), vitamin E and/or its derivatives (e.g., tocopherol acetate), vitamin B₆, vitamin B₁₂, biotin and/ or co-enzyme Q10.

The topical cosmetic compositions of the invention can also contain usual cosmetic or pharmaceutical adjuvants and additives, such as preservatives, film forming agents, fatty substances/ oils and/ or waxes, water, organic solvents, silicones, thickeners, softeners, emulsifiers, antifoaming agents, aesthetic components such as fragrances, surfactants, fillers, sequestering agents, anionic, cationic, nonionic or amphoteric polymers or mixtures thereof, propellants, acidifying or basifying agents, complexing agents, colorings/colorants, abrasives, absorbents, essential oils, skin sensates, astringents, perfumes or any other ingredients usually formulated into cosmetic compositions such as alcohols, polyols or electrolytes. Such cosmetic ingredients commonly used in the skin care industry, which are suitable for use in the compositions of the present invention, are e.g. described in the CTFA Cosmetic Ingredient Handbook, Second Edition (1992) without being limited thereto.

The necessary amounts of the cosmetic and pharmaceutical adjuvants and additives can - based on the desired product form- easily be chosen by a skilled person in this field and will be illustrated in the examples, without being limited hereto.

The usual cosmetic adjuvants and additives such as e.g. emulsifiers, thickeners, surface active ingredients and film formers can show synergistic effects which can be determined by the expert in the field with normal trials, or with the usual considerations regarding the formulation of cosmetic composition.

The fatty substances can be an oil or a wax, or mixtures thereof. By the term "oil" is intended a compound which is liquid at ambient temperature. By the term "wax" is intended a compound which is solid or substantially solid at ambient temperature and for which the melting point is generally greater than 35° C.

Exemplary oils are mineral oils (liquid paraffin); vegetable oils (sweet almond, macadamia, blackcurrant seed or jojoba oil); synthetic oils, such as perhydrosqualene, fatty alcohols, acids or esters (such as the C₁₂₋₁₅ alkyl benzoate marketed under the trademark "Finsolv TN" by Finetex, octyl palmitate, isopropyl lanolate or triglycerides, including those of capric/caprylic acids), or oxyethylenated or oxypropylenated fatty esters and ethers; silicone oils (cyclomethicone, polydimethylsiloxanes or PDMS); fluorinated oils; polyalkylenes and their mixtures.

Preferably the oils used in the compositions according to the invention are selected from the list of polar oils such as the lecitines and fatty acid triglycerides, namely triglycerinester of saturated or unsaturated, branched or linear alkanoic acids with a chain length of 8 to 24, particularly 12 to 18C-atoms. The fatty acid triglycerides may preferably be selected from the group of synthetic, semi synthetic and natural oils such as e.g. cocoglyceride, olive oil, sunflower oil, soy bean oil, peanut oil, palm oil, sweet almond oil macadamia oil, coconut oil etc.

Further particularly suitable are natural waxes such as bees wax, shea butter, and/ or lanolin. Further particularly suitable polar oils according to the present invention may be selected from the group of esters of saturated or unsaturated, branched or linear alkanoic acids with a chain length of 3 to 30 C-atoms and saturated or unsaturated, branched or linear alcohols with a chain length of 3 to 30C-atoms as well as from the group of esters from aromatic carbonic acids and saturated or unsaturated, branched or linear alcohols with a chain length of 3 to 30C-atoms. Such ester oils are particularly selected from the group of phenylethylbenzoate, octylpalmitate, octylcocoate, octylisostearate, octyldodeceylmyristate, octyldodecanol, cetearylisononanoate, isopropylmyristate, isopropylpalmitate, isopropylstearate, isopropyloleate, n-butylstearate, n-hexyllaurate, n-decyloleate, isooctylstearate, isononylstearate, isononylisononanoate, 2-ethylhexylpalmitate, 2-ethylhexyllaurate, 2-hexyldecylstearate, 2-octyldodecylpalmitate, stearylheptanoate, isopropyl lauroyl sarkosinate, oleyloleate, oleylerucate, erucyloleate, erucylerucate, tridecylstearate, tridecyltrimellitate as well as synthetic and semi synthetic and natural mixtures of such esters such as e.g. jojoba oil.

Further particularly suitable oils may be selected from the group of dialkyl ether and dialkylcarbonates such as particularly dicaprylylether (Cetiol OE) and/ or dicaprylylcarbonate, (e.g. available as Cetiol CC at Cognis).

Further particularly suitable oils may be selected from the group of isoeikosan, neopentylglykoldiheptanoate, propylenglykoldicaprylaet caprylate/dicaprate, caprylic/ capric/ diglyceryl succinate, butylene glyckol dicaprylate/dicaprate, C₁₂₋₁₃-Alkyllactate, Di-C₁₂₋₁₃-alkyltartrate, triisostearin, dipentaerythrityl hexacaprylate hexacaprate, propylenglykolmonoisostearate, tricaprylin and dimethylisosorbid.

It is particularly advantageous if the oil phase of the topical compositions according to the invention contains an amount of C₁₂₋₁₅-alkylbenzoate or consists essentially thereof.

Further particularly suitable oily components are e.g. butyloctylsalicylate (e.g. Hallbrite BHB from CP Hall), hexadecylbenzoate and butyloctylbenzoate as well as mixtures therof (e.g. Hallstar AB).

The topical compositions according to the present invention may also contain apolar oils such as e.g. branched or linear hydrocarbons and waxes, in particular mineral oil, vaseline (Petrolatum), paraffin oil, squalan and squalen, polyolefins, hydrogenated polyisobutenes, C₁₃₋₁₆ isoparaffin and isohexadecan. Within the group of polyolefins polydecenes are preferred.

Exemplary waxy compounds in particular suitable for the use in the compositions according to the invention are paraffin wax, carnauba wax, beeswax or hydrogenated castor oil.

Exemplary organic solvents in particular suitable for the use in the compositions according to the invention include the lower alcohols and polyols having at most 8 carbon atoms. In particular the compositions according to the invention comprise ethanol in an amount of 5 to 40 wt.-% based on the total weight of the composition.

The thickeners are advantageously selected, in particular, from among the cross linked polyacrylic acids or modified or unmodified guar gums and celluloses, such as hydroxypropylated guar gum, methylhydroxyethylcellulose and hydroxypropylmethylcellulose.

Suitable film forming agents include polymers on the basis of PVP such as in particular copolymers of polyvinylpyrrolidon e.g. PVP hexadecen copolymer and PVP eicosen copolymer which are available as Antaron V216 and Antaron V220 at GAF Chemicals corporations. Further suitable film forming agents include polymeric film formers such as sodiumpolystyrenesulfonate (e.g. Flexan 130 from National Starch and Chemical Corp.) and/ or polyisobuten (e.g. Rewopal PIB1000 from Rewo). Further suitable polymers are e.g. polyacrylamide (Seppigel 305), polyvinylalkohole, PVP, PVP/VA copolymers, polyglycols and acrylate/octylacralymid copolymers (e.g. Dermacryl 79). Further suitable is the use of hydrated castor oil dimerdilinoleat (CAS 646054-62-8, INCI Hydrogenated Castor Oil Dimer Dilinoleate), or PPG-3 Benzylethermyristate (CAS 403517-45-3).

The topical compositions according to the invention may further comprise one or several compounds from the group of siloxanes elastomers listed in order to enhance the water resistance and/ or enhance the light protection factor such as in particular siloxanes elastomers in the form of spherical powders with the INCI nomenclature Dimethicone/Vinyl Dimethicone Crosspolymer, such as e.g. DOW CORNING 9506 Powder (by Dow corning).

It is particularly advantageous if the siloxane elastomer is used in combination with hydrocarbon oils, synthetic oils, synthetic esters, synthetic ether or mixtures thereof.

Of course, one skilled in this art will take care to select the above mentioned optional additional compound or compounds and/or their amounts such that the advantageous properties intrinsically associated with the combination in accordance with the invention are not, or not substantially, detrimentally affected by the envisaged addition or additions.

Preferred topical compositions according to the invention are skin care preparation such as particularly skin-tanning preparations (i.e. compositions for the artificial/sunless tanning and/or browning of human skin).

Examples of skin care preparations are, in particular body oils, body lotions, body gels, treatment creams, skin protection ointments, moisturizing gels or moisturizing sprays,

The topical compositions are applied at least several times per week, preferably at least once per day, and more preferably applied at least twice a day such as e.g. once in the morning and once in the evening. Applications should be for a chronic period of time, i.e. at least one week, preferably for at least two weeks, and more preferably for at least 4 weeks in order to observe results.

The topical compositions according to the present invention can also be used to achieve a cosmetic effect, which could be beautifying skin, in particular the treatment or prophylaxis of wrinkles or dry skin or sensitive skin or any symptoms caused by negative developments of the physiological homeostasis of healthy skin, moisturizing the skin, thickening of the epidermis, treatment or prophylaxis of acne, inhibition of senescence of skin cells, prevention or treatment of photodamage, prevention or treatment of oxidative stress phenomena, prevention or treatment of cellulite, prevention or treatment of pigmentation disorders and/or to even and/or darken the skin tone, prevention and treatment of disturbances in ceramide and lipid synthesis, prevention of excess sebum production, reduction of activities of matrix metallo proteases or other proteases in the skin, treatment and prevention of inflammatory skin conditions including atopic eczema, polymorphic light eruption, psoriasis, vertiligo, prevention and treatment of itchy or irritated skin, strengthening and/or protection of nails or a skin cleansing effect.

The invention is further illustrated by the Examples which follow without being limited thereto.

### Example 1: Induction of melanin synthesis by Steviol in human epidermal melanocytes Quantification of melanin synthesis in cell culture:

Normal human melanocytes NHM (HEMn-MP, Clonetics) were seeded in 96 well cell culture plates and grown to sub-confluence for two days in a mixture of M2-Medium (Clonetics) and Medium (Promocell). Culture medium was exchanged with Culture Medium containing Steviol (96%) and melanogenesis progressed for another three days at 37°C with another medium exchange on day two. Including cell layer and culture supernatant the total melanin was extracted using 1.7M KOH with vigorous shaking at RT. The total melanin content was determined at 405 nm in an absorbance plate reader versus a control (100%)
The results are summarized in the table below:

| Control | Positive control Glycyrrhizin | Steviol | | |
|---|---|---|---|---|
| | 1.5 mM | 0.25 microM | 0.5 micro M | 1 micro M |
| 100% | 132% | 109% | 124% | 130% |

Glycyrrhizin is used as a positive control since it is a known inducer of melanogenesis. Steviol shows a clear dose dependent positive effect on the production of melanin in human melanocytes. Furthermore, as can be retrieved from the results, steviol is about 1000 times more potent than the positive control Glycyrrhizin.

### Example 2: Discoloration experiment

Steviol (87%) was dissolved at 1 wt.-% in water and the pH adjusted to different values as shown below. At pH 9, the solution turns brown within 1 day. However, the more acidic the pH, the less discoloration was observed. The color was determined visually using the Pantone Color Index.

Pantone Colours index of 1% Steviol solution in water at different pH:

| | **pH 9** | **pH 7** | **pH 6.5** | **pH 6** |
|---|---|---|---|---|
| **Pantone Index** | **160** | **158** | **155** | **155** |

As can be retrieved from the results presented above, a significant reduction of the discoloration can be achieved at by lowering the pH.

### Example 3

The following formulation examples are provided to further illustrate the use of the compounds and compositions of the present invention. These examples are illustrative only and are not intended to limit the scope of the invention in any way. The respective formulations are prepared according to methods known to a person skilled in the art.
The names of the ingredients in the following tables are indicated as INCI names. All amounts are given as wt.-% based on the total weight of the composition.

| **Gels** | **1** | **2** | **3** | **4** | **5** | **6** | **7** | **8** |
|---|---|---|---|---|---|---|---|---|
| Acrylates/Octylacrylamide Copolymer | 1 | 1 | 1 | 1 | 1 | 1 | 1 | 1 |
| Alcohol Denat. | 50 | 62 | 59.2 | 52 | 56 | 59 | 51 | 54 |
| Butylene Glycol Dicaprylate/Dicaprate | | | 7.5 | | | 2 | 4 | |
| C12-15 Alkyl Benzoate | 5 | 8.5 | 5 | 2 | 7.5 | | 2 | 5 |
| Phenylethylbenzoate | 3 | | | 2.5 | | | 2.5 | |
| Cocoglyceride | | | | | | 2 | 5 | |
| Tridecylsalicylate | 2 | 1.5 | | | 3 | 1 | | 3 |
| Hydroxypropoylcellulose | 2 | 0.8 | 1 | 0.8 | 0.5 | 0.8 | 0.45 | 0.5 |
| Butyl Methoxydibenzoylmethane | 4.5 | | 2.5 | | 2.5 | 4.5 | | 3 |
| Merocyanine | | | | | 1.8 | | | 5 |
| 2,4,6-Tribiphenyl-4-yl-1,3,5 Triazine | | | 0.5 | 4 | | 6 | | |
| Diethylamino Hydroxybenzoyl Hexyl Benzoate | | | 4.5 | 3.5 | | | | |
| Ethylhexyl Methoxycinnamate | | | | 6.5 | 6.5 | | | |
| Ethylhexyl Salicylate | 3 | | 4.5 | 4.5 | | | | 5 |
| Homosalate | | | | | | 4.5 | | |
| Octocrylene | 8 | | 5.5 | 4.3 | 3.8 | | | 4 |
| Ethylhexyl Triazone | | | | | 2 | | | |
| Benzophenone-3 | 3 | | | | | | | |
| Drometrizole Trisiloxane | | | | 0.5 | 1 | | | 1 |
| Steviol | 0.8 | 0.15 | 0.25 | 0.05 | 0.35 | 0.2 | 0.02 | 0.15 |
| Bis-Ethylhexyloxyphenol Methoxyphenyltriazine | | | | | | 1 | | |
| Benzotriazoyl Dodecyl p-Cresol | | | | | | 5 | | |
| Butyloctyl Methoxycrylene | | | 4 | | | | | |
| Diethylhexyl Syringylidenemalonate | | | | | | | | |
| Vitamin E Acetate | | | | 0.5 | | 0.2 | 0.5 | |
| Glycerin | 5 | | 3 | | | | | |
| Ascorbylglucoside | | 0,1 | | 0,5 | | | 1,0 | |
| Fragrance, Colours | q.s | | | | | | | |
| Water | ad 100 | | | | | | | |

| **Sprays** | **1** | **2** | **3** | **4** | **5** | **6** | **7** | **8** | **9** | **10** | **11** | **12** |
|---|---|---|---|---|---|---|---|---|---|---|---|---|
| Acrylates/Octylacrylamide Copolymer | 1 | | | | 1 | 1 | | | 1 | | 1 | |
| VP/VA Copolymer | | 1 | | 0.5 | | | | 0.5 | | | | 0.5 |
| Alcohol Denat. | 42 | 57 | 60 | 53 | 35.5 | 43.5 | 40 | 53 | 35.5 | 20 | 34 | 53 |
| Potassium Cetyl Phosphate | | | | | | | | | | 2 | 3 | |
| Cetearyl Alcohol | | | | | | | | | | 0.2 | | |
| Cetyl Alcohol | | | | | | | | | | | 0.3 | |
| Acrylates/C10-30 Alkyl Acrylate Crosspolymer | | | | | | | | | | 0.2 | 0.15 | |
| Cyclomethicone | 4.9 | 2 | 5 | 0.5 | 8 | 8 | 3 | 0.5 | 10 | 4 | 10 | 0.5 |
| Tridecylsalicylate | 0.5 | 2.5 | 4 | 7 | 10 | 3 | 3 | 7 | 4 | 0.5 | 4 | 7 |
| Bis-Ethylhexyloxyphenol Methoxyphenyltriazine | | | | | 1 | | | | 1 | | 1 | |
| Ethylhexyl Bis-Isopentylbenzoxazolyl phenyl Melamine | 0.5 | | 1 | | | 3 | | | | | | |
| Butyl Methoxydibenzoylmethane | 4.5 | 2 | | 3 | 5 | | | 3 | 3-5 | 4 | 3 | 3 |
| Diethylamino Hydroxybenzoyl Hexyl Benzoate | | 2 | 3 | | | 4.5 | | | | | | |
| Ethylhexyl Methoxycinnamate | | | | 7 | | 8.5 | | 7 | | 7.5 | | |
| Ethylhexyl Salicylate | 4.5 | 3.5 | 2 | 4 | 4.5 | 1.5 | | 4 | 5 | | 4 | 4 |
| Drometrizole Trisiloxane | | 0.5 | | | | 1 | | | | 2 | | |
| 2,4,6-Tribiphenyl-4-yl-1,3,5 Triazine | 1 | | | 5 | | | | 2 | | | | 2 |
| Merocyanine | | | 3 | | | 0.5 | | | | 2 | | |
| Methylene Bis-Benztriazoyl Tetramethylbutylphenol | | | | | | | | 3 | | | | |
| 1,1'-(1,4-piperazinediyl)bis[1-[2-[4-(diethylamino)-2-hydroxybenzoyl] phenyl]-methanone | | | | | | | | | | | | 3 |
| Homosalate | 9.5 | 5 | 3 | | | 5.5 | | | 15 | 5 | | 7 |
| Octocrylene | 9.5 | | 8 | | 7.5 | 8.5 | | | 10 | | | |
| Steviol | 0.3 | 0.2 5 | 0.6 8 | 0.1 | 0.4 | 0.02 | 0.2 | 0.1 | 0.3 | 0.1 5 | 2 | 1 |
| Phenylbenzimidazole Sulfonic Acid | | | | | 2 | | | | 0-3 | | | |
| Polysilicone-15 | | 1.5 | | | 2 | | | | 0.99 | | | |
| Methylbenzylidene Camphor | | 1 | | | | | | | | | | |
| Butylene Glycol Dicaprylate/Dicaprate | | 9 | | | | | | | | 2 | | |
| C₁₂₋₁₅ Alkyl Benzoate | | 2 | | 2 | | | | 2 | 5 | | 5 | 2 |
| Phenylbenzoate | | | 7 | | 4 | | 7 | | 4 | | 4 | |
| Benzotriazoyl Dodecyl p-Cresol | | | | | | | | 3 | | | | 3 |
| Butyloctyl Methoxycrylene | | | | | | | | | | 4 | | |
| Diethylhexyl Syringylidenemalonate | | 2 | | | | | | | | | | |
| Diethylhexylnaphthalate | | 6 | | 3 | | | | 3 | | | | 3 |
| Isopropyl Lauroyl Sarcosinate | 2 | | 1 | | 3 | | 1 | | 3 | | 3 | |
| Phenyl Trimethicone | 2 | 5 | | | 1 | 2 | | | 1 | 2 | 1 | |
| Octyldodecanol | | | | 8 | | | | 8 | | | | 8 |
| Glycerin | 5 | 4 | 5 | 8 | 5 | 5 | 5 | 8 | 5 | 5 | 5 | 8 |
| Vitamin E Acetate | 0.1 | | 0.5 | | | | 0.5 | | | | | |
| Fragrance, Colours | q.s | | | | | | | | | | | |
| Water | Ad 100 | | | | | | | | | | | |

| **O/W Emulsions** | **1** | **2** | **3** | **4** | **5** | **6** | **7** | **8** | **9** | **10** |
|---|---|---|---|---|---|---|---|---|---|---|
| Glyceryl Stearate Citrate | 2 | 2 | 3 | | | | | | | 3 |
| Glyceryl Stearate SE | | | | 1 | 1 | 1.5 | | | 1.5 | |
| Cetearyl Alcohol + PEG-40 Rizinusoil + Sodium Cetearyl Sulfate | | | | 2.5 | 2.5 | 3 | | | | |
| Potassium Cetyl Phosphate | | | | | | | 2 | 2 | 1.5 | |
| Cetearyl Alcohol | | | 1 | 1 | | | 2 | 2 | 0.5 | 1 |
| Stearyl Alcohol | 0.5 | | | | | 2 | | | 0.5 | |
| Myristyl Myristate | 1 | 1 | | | 3 | | | 2 | | |
| Acrylates/C₁₀₋₃₀ Alkyl Acrylate Crosspolymer | 0.1 | 0.2 | | | 0.1 | | | 0.2 | | |
| Carbomer | | 0.2 | 0.3 | 0.2 | | | | | | 0.3 |
| Xanthan Gum | 0.4 | | 0.2 | 0.2 | 0.3 | 0.4 | 0.2 | | 0.3 | 0.2 |
| C₁₂₋₁₅ Alkyl Benzoate | | 3 | | | 5 | | 4 | 5 | | |
| 2-Phenylethylbenzoate | 5 | 2 | | | | | | | | |
| Butylene Glycol Dicaprylate/Dicaprate | 5 | | | | 3 | 3 | | 7.5 | 3 | |
| Tridecylsalicylate | 1.5 | 2.5 | 0.25 | 5.9 | 7 | 15 | 5.9 | 7 | 6 | 0.25 |
| Dicaprylcaprate | 2 | 2 | | | 2 | 2 | | 2 | 2 | |
| Cyclomethicone | | | | 5 | 10 | | 5 | | | |
| Dimethicone | | | | | 5 | | | 5 | | |
| PVP Hexadecene Copolymer | | 0.5 | | | | 1 | 2 | | 1 | |
| Propylene Glycol | | | 1 | | 5 | 3 | | | 3 | 1 |
| Ascorbylglucoside | | 0,1 | | | 0,5 | | | | 1,0 | |
| Glycerin | 3 | 5 | 7 | 10 | 13 | 3 | 3 | 5 | 3 | 7 |
| Alcohol denat. | 2 | 3 | | 7 | | | | | | |
| Merocyanine | 1 | | | | 3 | | 0.8 | | | |
| Titanium Dioxide | 3 | | | 2 | | | 3 | | | |
| Phenylene-1,4-bis-(2-benzimidazyl)-3,3-5,5-tetrasulfonic Acid | 3 | 2 | | | | | | | | |
| Ethylhexyl Triazone | 2.5 | 2 | | 1 | | | 1 | | | |
| Phenylbenzimidazole Sulfonic Acid | | | 2 | | | 1 | 2 | | 1 | 2 |
| 4-Methoxycinnamate (2-ethylhexyl) ester | | 5 | | 2 | | | | | | |
| Butyl Methoxydibenzoylmethane | 5 | 1 | | | 3 | | 4 | | 5 | |
| Ethylhexylsalicylate | 5 | | 0.5 | 4 | 5 | | 4 | | | 0.5 |
| Polysilicone-15 | | | 4 | | | | 1 | | | 4 |
| Isoamyl p-Methoxycinnamate | | 3 | 6 | | | | | | | 6 |
| Diethylamino Hydroxybenzoyl Hexyl Benzoate | | | | 6 | | | | | 3 | |
| Methylene Bis-Benztriazoyl Tetramethylbutylphenol | | | 5 | | | | | | | |
| 1,1'-(1,4-piperazinediyl)bis[1-[2-[4-(diethylamino)-2-hydroxybenzoyl] phenyl]-methanone | | | | | | | | | | 5 |
| Octocrylene | 3 | | | | 5 | 7 | 4 | | 7 | |
| Bis-Ethylhexyloxyphenol Methoxyphenyltriazine | | 1 | | 1 | | 1 | 0.5 | | 1 | |
| Steviol | 0.01 | 0.1 | 0.4 | 0.6 | 0.3 | 0.5 | 2 | 1 | 1.5 | 0.4 |
| Benzotriazoyl Dodecyl p-Cresol | | 0.9 | | | | | | | | |
| Butyloctyl Methoxycrylene | | | | | 3 | | | | | |
| Diethylhexyl Syringylidenemalonate | | | | | | | 2 | | | |
| Vitamin E Acetat | 0.2 | 0.2 | 0.2 | 0.3 | 0.1 | 0.5 | 0.3 | 0.1 | 0.5 | 0.2 |
| Disodium EDTA | 0.1 | 0.1 | 0.2 | 0.2 | 0.5 | 0.2 | 0.2 | 0.2 | 0.2 | 0.2 |
| DHA | | | | | | | 2 | 0.8 | | |
| Erythrulose | | | | 1.9 | | | | 0.4 | | |
| Fragrance, Preservation agents | q.s | q.s | q.s | q.s | q.s | q.s | q.s | q.s | q.s | q.s |
| Colours, etc. | q.s | q.s | q.s | q.s | q.s | q.s | q.s | q.s | q.s | q.s |
| Citric Acid, Sodium Citrate | q.s | q.s | q.s | q.s | q.s | q.s | q.s | q.s | q.s | q.s |
| Sodium Hydroxide | q.s | q.s | q.s | q.s | q.s | q.s | | q.s | | q.s |
| Tromethamine | | | | | | | q.s | | q.s | |
| Water | ad 100 | | | | | | | | | |

| **O/W Emulsions** | **11** | **12** | **13** | **14** | **15** | **16** | **17** | **18** | **19** |
|---|---|---|---|---|---|---|---|---|---|
| Glyceryl Stearate | 2.5 | 2 | 1.2 | 1 | | | 1 | 1 | |
| PEG-40 Stearate | 1 | | | | | | | | |
| PEG-100 Stearate | | 2.5 | | | | | | 1 | |
| Ceteareth-20 | | | | | 1 | | | | |
| Glyceryl Stearate Citrate | | | | | | 0.5 | | | 0.5 |
| Potassium Cetyl Phosphate | | | | | | | 3 | 1.5 | 2 |
| Stearic Acid | | | 2.5 | 3 | | | | | |
| Cetearyl Alcohol | 4 | | | 2 | | | 2 | | |
| Stearyl Alcohol | | 2 | 1 | | | | | | |
| Cetyl Alcohol | | | 1 | 1 | | | | 0.5 | |
| Acrylates/C₁₀₋₃₀ Alkyl Acrylate Crosspolymer | | | | 0.2 | 0.2 | 0.4 | 0.2 | | 0.4 |
| Carbomer | 0.1 | | 0.2 | | | | | | |
| Xanthan Gum | | 0.3 | | | | | | 0.3 | |
| C₁₂₋₁₅ Alkyl Benzoate | 5 | | | 2 | 5 | 5 | 10 | 5 | 5 |
| Vaseline | 5 | | 3 | | | | | | |
| Butylene Glycol Dicaprylate/Dicaprate | | 4 | 2 | | 9 | | | 9 | |
| Hydrogenated Polydecene | | | 3 | | 2 | | | 2 | |
| Caprylic/Capric Triglyceride | 1 | 3 | | 5 | | 5 | 5 | | 5 |
| Cyclomethicone | | 5 | 2 | | | 10 | | | 10 |
| Methylpropandiol | 2 | | | | 3 | | | 3 | |
| Glycerine | 7.5 | 10 | 4 | 5 | 5 | | 5 | 5 | |
| Alcohol denat. | 1 | 3 | 0.5 | 10 | 4 | 8 | | 4 | 8 |
| Butylene Glycol | | | 3 | | | | | | |
| Ascorbylglucoside | | 0,5 | | 1,0 | | 1,5 | | 0,1 | |
| Isotridecylsalicylate | | | 1 | 3 | 5 | 2 | 3 | 5 | |
| Titanium Dioxide | 1 | | 0.5 | 2 | | | | | 5 |
| Merocyanine | | | | | 1.8 | | | 5 | |
| 2,4,6-Tribiphenyl-4-yl-1,3,5 Triazine | | | 0.5 | 4 | | 6 | | | |
| Ethylhexyl Bis-Isopentylbenzoxazolylphenyl Melamine | | 1 | | | 0.5 | | | 2 | |
| Ethylhexyl methoxycinnamate | | | | | 2 | | | | |
| Phenylbenzimidazole Sulfonic Acid | 1.5 | | | 2 | | 2 | 0-2 | | |
| Butyl Methoxydibenzoylmethane | 2.5 | 1 | | 2 | 2 | 3 | 3-5 | 3 | |
| Methylbenzylidene Camphor | | | | | 2 | 3 | | | |
| Disodium Phenyl Dibenzimidazole Tetrasulfonic Acid | | | | | | | | | 2 |
| Steviol | 0.15 | 0.1 | 0.4 | 0.3 | 0.5 | 0.3 | 0.03 | 2 | 1.6 |
| Octocrylene | | 5 | | | | 2 | 10 | | 2 |
| Polysilicone-15 | | | | 2 | | 3 | | | |
| Ethylhexyl Salicylate | 3 | | | | 5 | | | | |
| Homosalate | | | 4 | | 2 | | | | 3 |
| Benzophenone-3 (Oxybenzone) | | | | | | | | | |
| Drometrizole Trisiloxane | 0.5 | | | 1 | | 2 | | | |
| Terephthalidene Dicamphor Sulfonic Acid | 1.5 | | | 0.5 | | 0.25 | | | |
| Benzotriazoyl Dodecyl p-Cresol | 3 | | | | | | | | |
| Butyloctyl Methoxycrylene | | | | | | 2 | | | |
| Tapioca Starch | 1 | | 2.5 | | | 0.5 | | | 0.5 |
| Sodium Starch Octenylsuccinate | | | | 1 | | | 1 | | |
| Disodium EDTA | 0.1 | | | | | 0.5 | | | 0.5 |
| Fragrance, Preservatives | q.s | q.s | q.s | q.s | q.s | q.s | q.s | q.s | q.s |
| Sodium Hydroxide | q.s | q.s | q.s | q.s | q.s | q.s | q.s | q.s | q.s |
| Water | Ad 100 | | | | | | | | |

| **W/O Emulsion** | **1** | **2** | **3** | **4** | **5** | **6** | **7** | **8** | **9** | **10** | **11** | **12** | **13** | **14** |
|---|---|---|---|---|---|---|---|---|---|---|---|---|---|---|
| Polyglyceryl-2 Dipolyhydroxystearate | 3 | 5 | 3 | | | | | | 5 | 5 | 5 | 3 | 3 | |
| PEG-30 Dipolyhydroxystearate | | | 2 | 3 | 4 | 5 | 3 | 4 | | | | | 2 | 4 |
| Sodium Starch Octenylsuccinate | 0.5 | 0.4 | 0.6 | 0.3 | 0.5 | 1 | 0.3 | 0.5 | 1 | 0.5 | 1 | 0.5 | 0.6 | 0.5 |
| Glycine | 0.3 | 0.3 | 0.5 | 0.4 | | | 0.4 | | | | | 0.3 | 0.5 | |
| Alcohol denat. | 2 | 5 | 2 | 0.5 | 8 | 1 | | 8 | 5 | | 3 | 2 | 2 | 8 |
| Magnesium Sulfate | 0.2 | 0.3 | 0.3 | 0.4 | 0.5 | 0.2 | 0.5 | 0.5 | 0.5 | 0.5 | 0.5 | 0.2 | 0.3 | 0.5 |
| C12-15 Alkyl Benzoate | 5 | 3 | | | 5 | | | 5 | | 4 | | 5 | | 5 |
| Triheptanoin | | 2 | | | | | | | | | | | | |
| Butyleneglycol Dicaprylat/Dicaprate | 5 | | | | 3 | 3 | | 3 | 3 | 6 | 3 | 5 | | 3 |
| Dicaprylyl Ether | | | | | 2 | | | 2 | | 2 | | | | 2 |
| Mineral Oil | | 4 | | 6 | | 8 | 6 | | 5 | | 8 | | | |
| Octyldodecanol | 2 | | | | | | | | | | | 2 | | |
| Dicapryl Caprate | | 2 | | | 2 | 2 | | 2 | 2 | 2 | 2 | | | 2 |
| Cyclomethicone | 5 | | 5 | 10 | | | | | | | | 5 | 5 | |
| Dimethicone | | | | 5 | | | 5 | | | | | | | |
| Isohexadecane | | 1 | | | | | | | | | | | | |
| Butylene Glycole | 5 | 8 | | | | 3 | | | 3 | | 3 | 5 | | |
| Glycerin | 3 | 5 | 7 | 10 | 3 | 3 | 10 | 3 | 3 | 3 | 3 | 3 | 7 | 3 |
| Tridecylsalicylate | | 1 | 2 | | | 0.5 | | | 0.5 | | 0.5 | | 2 | |
| 2-Phenylethylbenzoate | | | 2 | | 4 | | | 4 | | | | | 2 | 4 |
| Isopropyl Lauroyl Sarcosinate | | | 1 | | 2 | | | 2 | | | | | 1 | 2 |
| Ethylhexylmethoxycinnamate | 2 | | | | | | | 5 | | | | 5 | 7 | 5 |
| Ethylhexyl Triazone | 2 | 3 | 3 | | | 3 | | | | 1 | | 2 | 3 | |
| Ethylhexyl Bis-Isopentyl-benzoxazolylphenyl Melamine | | | | 3 | | | | 0.5 | | | | | | |
| Diethylhexyl Butamido Triazone | | 1.5 | 1.5 | | | 1.5 | | | | | | | 1.5 | |
| Butyl Methoxy dibenzoylmethane | 1 | 4 | 3 | | 5 | 2.5 | 2 | 5 | | 2 | 3 | | | |
| Methylbenzylidene Camphor | | | | | 1 | | | | | | 2 | | | |
| 2,4,6-Tribiphenyl-4-yl-1,3,5 Triazine | | | 5 | | | | | 2 | | | | | 5 | |
| Merocyanine | | | | | 3 | | | | | 2 | | | | 3 |
| 2-(4-Diethylamino-2-hydroxybenzoyl)-Benzoic Acid Hexylester | | | | 2 | | | | 1 | | | | | | |
| Steviol | 0.2 | 0.4 | 0.3 | 0.075 | 0.5 | 0.25 | 1 | 1.5 | 2 | 1.5 | 1 | 0.2 | 0.4 | 0.5 |
| Titanium Dioxide (Parsol TX) | 5 | 4 | 2 | | 3 | 4 | | 3 | | 3 | 4 | 5 | 2 | 3 |
| Polysilicone-15 | 2 | | | | 1.5 | 2 | | | | 3 | | | | |
| Octocrylene | | 3.6 | | | | 2 | | 5 | | 2 | | | | |
| Ethylhexyl Salicylate | | | | | 5 | | | | | | | | | |
| Phenylbenzimidazole Sulfonic Acid | 3 | | | | | | 1 | | | 2 | | | | |
| Bis- Ethyl hexyloxyphenol Methoxyphenyltriazine | 1 | 2 | 2 | | 2 | 3 | | | | 1.5 | | 1 | 2 | 2 |
| Methylene Bis-benzotriazolyl tetramethylbutylphenol | 2 | | 3 | | 1 | | | | | | 1 | | | |
| 1,1'-(1,4-piperazinediyl)bis[1-[2-[4-(diethylamino)-2-hydroxybenzoyl] phenyl]-methanone | | | | | | | | | | | | 2 | 3 | 1 |
| Benzotriazoyl Dodecyl p-Cresol | | | | | | | | | | | 3 | | | |
| Butyloctyl Methoxycrylene | | 4 | | | | | | | | | | | 4 | |
| Vitamin E Acetate | 0.2 | 0.2 | 0.2 | 0.3 | 0.1 | 0.5 | 0.3 | 0.1 | 0.5 | 0.1 | 0.5 | 0.2 | 0.2 | 0.1 |
| Ascorbylglucoside | 0,2 | | 0,5 | | | | 1,0 | | | 2,0 | | | | |
| Disodium EDTA | 0.1 | 0.1 | 0.2 | 0.2 | 0.2 | 0.5 | 0.2 | 0.2 | 0.5 | 0.2 | 0.5 | 0.1 | 0.2 | 0.2 |
| Fragrance, Preservatives | q.s | q.s | q.s | q.s | q.s | q.s | q.s | q.s | q.s | q.s | q.s | q.s | q.s | q.s |
| Water | ad 100 | | | | | | | | | | | | | |

| **Hydrodispersions** | **1** | **2** | **3** | **4** | **5** | **6** | **7** | **8** |
|---|---|---|---|---|---|---|---|---|
| Glyceryl Stearate Citrate | | 0.4 | | | | | | |
| Sodium Carbomer | | | | | 0.3 | | | |
| Acrylates/C₁₀₋₃₀ Alkyl Acrylate Crosspolymer | | | 0.3 | 0.4 | 0.1 | 0.1 | 0.2 | 0.1 |
| Ceteareth-20 | | | 1 | | | | | |
| Potassium Cetyl Phosphate | | | | | 2 | | | 2 |
| Xanthan Gun | 0.5 | | | 0.15 | | 0.5 | 0.2 | 0.2 |
| Dimethicone/Vinyl Dimethicone Crosspolymer | | | | 5 | | 3 | 1.5 | |
| 2-(4-Diethylamino-2-hydroxybenzoyl)-benzoic Acid Hexylester | | | | 0.5 | 2 | 1.5 | | 1.5 |
| 2,4,6-Tribiphenyl-4-yl-1,3,5 Triazine | | | | | 3 | | 0.5 | |
| Merocyanine | | 2 | | | | | | 4 |
| Butyl Methoxydibenzoylmethane | 2 | | 3.5 | 2 | 3 | | 5 | 2 |
| Ethylhexyl Bis-Isopentylbenzoxazolylphenyl Melamine | | | 0.5 | | | 2 | | |
| Bis-Ethylhexyloxyphenol Methoxyphenyltriazine | | 2 | | 0.25 | | | | 1 |
| Disodium Phenyl Dibenzimidazole Tetrasulfonate | 2 | | | | | 1 | | |
| Phenylbenzimidazole Sulfonic Acid | | 1 | 2 | | | | | 0.5 |
| Ethylhexyl Methoxycinnamate | | | | | | 8 | | |
| Ethylhexyl Salicylate | | | | | 4 | | | |
| Homosalate | | | | | 10 | | | |
| Diethylhexyl Butamido Triazone | | | 2 | 2 | | | | |
| Ethylhexyl Triazone | 4 | 3 | | | 4 | | 1 | 1 |
| Octocrylene | 2 | | | 1 | | | | |
| Polysilicone-15 | | 0.9 | | | | | | 3 |
| Methylbenzylidene Camphor | | | 3 | | | | 2 | |
| Steviol | 0.01 | 0.5 | 0.3 | 0.3 | 0.3 | 0.8 | 0.1 | 0.15 |
| Titanium Dioxide | 0.5 | 2 | 1 | 2 | 0-3 | 1 | 2 | |
| Drometrizole Trisiloxane | | | | 1 | | 0.5 | | |
| Terephthalidene Dicamphor Sulfonic Acid | | | | 0.5 | | 0.75 | | |
| Benzotriazoyl Dodecyl p-Cresol | | | 3 | | | | 8 | |
| C₁₂₋₁₅ Alkyl Benzoate | 2 | | 2.5 | | | | | 7.5 |
| Butylene Glycol Dicaprylate/Dicaprate | 4 | | | | 6 | | | |
| Dicaprylyl Carbonate | | 3 | | | | | | 1.5 |
| Dicaprylylether | | 2 | | | | | | |
| Cyclomethicone | | | | 7.5 | | | 3 | |
| 2-Phenylethylbenzoate | | | 4 | | 2 | | | |
| Diethylhexylnaphthalate | 5 | | 6 | | | | | |
| Tridecylsalicylate | 2 | 3 | 1 | 5 | 3 | 0.5 | 3 | |
| PVP Hexadecene Copolymer | 0.5 | | 0.5 | | 0.5 | 1 | | |
| Glycerin | 10 | 5 | 5 | | 5 | 8 | | 3 |
| Butylene Glycol | | 7 | | | | | | |
| Glycine Soja | | | | 1 | | | 1 | |
| Vitamin E Acetate | 0.5 | 0.25 | 0.5 | 0.25 | 0.75 | 1 | 0.25 | 0.5 |
| Alpha-Glycosylrutin | | | | | 0.25 | | | |
| DHA | | 1.2 | | | | 1.0 | | |
| Erythrulose | 0.5 | | | | | 0.5 | | |
| Trisodium EDTA | | 0.1 | 0.1 | 0.1 | 0.2 | | 0.1 | 0.1 |
| Tromethamine | | | q.s. | | | | | q.s. |
| Ethanol | 3 | 10 | 4 | 3.5 | 0.5 | 1 | | |
| Preservatives | q.s | q.s | q.s | q.s | q.s | q.s | q.s | q.s |
| Fragrance, Colours | q.s | q.s | q.s | q.s | q.s | q.s | q.s | q.s |
| Water | Ad 100 | | | | | | | |

| **Foams** | **1** | **2** | **3** | **4** | **5** | **6** |
|---|---|---|---|---|---|---|
| Stearic Acid | 2 | 2 | | | | |
| Palmitic Acid | | | 1.5 | | 1.5 | 1.5 |
| Cetyl Alcohol | 2.5 | 2 | | 2 | | |
| Potassium Cetyl Phosphate | | | | 2 | 1.5 | 1.5 |
| Stearyl Alcohol | | | 3 | | 3 | 3 |
| PEG-100 Stearate | | | 3.5 | | | |
| PEG-40 Stearate | | 2 | | | | |
| PEG-20 Stearate | 3 | | | | | |
| Sorbitan Stearate | | 0.8 | | 0.5 | | |
| C₁₂₋₁₅Alkyl Benzoate | 5 | | | | | 8 |
| C₁₂₋₁₃Alkyl Tartrate | | | 7 | | 7 | |
| Butyleneglycol Dicaprylate/Dicaprate | | 6 | | 5 | | |
| Dicaprylyl Ether | | | 2 | | 2 | 2 |
| Cyclomethicone | | 2 | 3 | | 3 | |
| Butylene Glycol | 1 | | | | | 3 |
| Isohexadecane | 2 | | | | | |
| Methylpropandiol | | | | | | |
| Propylene Glycol | | | 5 | | 5 | |
| Glycerin | 5 | 7 | | 3 | | |
| 2-(4-Diethylamino-2-hydroxybenzoyl)-Benzoic Acid Hexylester | 2 | | | | | |
| Merocyanine | | | | | | 2.4 |
| Butyl Methoxydibenzoylmethane | 3 | 2 | | 3 | | 4 |
| Dimethicodiethylbenzalmalonate | | 3 | | | | |
| Homosalate | | 5 | | | | 5 |
| Phenylbenzimidazole Sulfonic Acid | | 2 | 2 | | | |
| Benzophenone-3 | 2 | | | | | |
| Ethylhexyl Salicylate | | 5 | | 3 | | |
| Octocrylene | 2 | | | 3 | | 2 |
| Bis-Ethylhexyloxyphenol Methoxyphenyltriazine | | 3 | | 1 | | |
| 2,2 Methylen-bis-(6(2H-benzotriazol-2-yl)-4-(1,1,3,3-tetramethylbutyl)phenol | | | 8 | | | |
| 2,4,6-Tribiphenyl-4-yl-1,3,5 Triazine | 5 | | 0.5 | | | |
| Steviol | 0.3 | 0.03 | 0.6 | 0.15 | 2 | 1 |
| C₈-C₁₆ Alkylpolyglycoside | 1 | | | | | |
| Vitamin E Acetate | 0.6 | 0.5 | 0.2 | 0.5 | 0.2 | 0.2 |
| Creatine/ Creatinine | | | 0.5 | | 0.5 | 0.5 |
| BHT | | | 0.1 | | 0.1 | 0.1 |
| Disodium EDTA | 0.5 | | | | | |
| Fragrance, Preservatives | q.s | q.s | q.s | q.s | q.s | q.s |
| Colours | q.s | q.s | q.s | q.s | q.s | q.s |
| Sodium Hydroxide | q.s | | | | q.s | q.s |
| Potassium Hydroxide | | | | q.s | | |
| Tromethamine | | q.s | q.s | | | |
| Water | ad 100 | | | | | |

### Example 4 Skin tanning preparations comprising Steviol

| **O/W-Emulsions** | **4.1** | **4.2** |
|---|---|---|
| | **Wt.-%** | **Wt.-%** |
| Cetearyl Alcohol + Sodium Cetylstearylsulfat | 3.00 | 2.00 |
| Glycerylstearat SE | 2.00 | 4.00 |
| Octyldodecanol | 2.00 | 2.00 |
| C12-15 Alkylbenzoate | 1.00 | 1.00 |
| C13-16 Isoparaffin | 3.00 | 3.00 |
| Caprylic acid -/Capric acid triglyceride | 2.00 | 2.00 |
| Glycerine | 5.00 | 6.00 |
| Dimethicone | 0.50 | 0.50 |
| Sodium ascorbylphosphate | 0.10 | - |
| Ethylhexylsalicylate | 0.50 | 0.50 |
| Glycyrrhetic acid | - | 0.10 |
| Steviol | 1.00 | 1.50 |
| Grape seed oil | 0.50 | 0.50 |
| Dihydroxyacetone | - | 2.00 |
| Paraffinum Liquidum + Ginkgo Biloba Extract | 0.25 | 0.25 |
| Citric acid | 0.09 | 0.09 |
| Sodium citrate | 0.17 | 0.17 |
| Xanthan gum | - | 0.10 |
| Ammonium Acryloyldimethyltaurate/VP Copolymer | 0.40 | - |
| Carbomer | - | 0.30 |
| Parabene | 0.30 | 0.30 |
| Phenoxyethanol | 0.50 | 0.50 |
| Alcohol Denat. | 3.50 | 3.50 |
| Perfume | q.s | q.s |
| Water | ad 100 | ad 100 |

| **Sprayable emulsions** | **4.3** | **4.4** |
|---|---|---|
| | **Wt.-%** | **Wt.-%** |
| Isoceteth-20 | 5.00 | 3.00 |
| Glycerylisostearate | 3.00 | 2.00 |
| Mineral Oil | 5.00 | 4.00 |
| Glycerine | 4.00 | 5.00 |
| Tocopherylacetate | 0.50 | 0.40 |
| Steviol | 0.20 | 0.50 |
| Natriumcitrate | 0.40 | 0.40 |
| Phenoxyethanol | 0.40 | 0.40 |
| Citric acid | 0.20 | 0.20 |
| DMDM Hydantoin | 0.20 | 0.20 |
| Perfume | q.s. | q.s. |
| Water | ad 100 | ad 100 |

The pH of the formulation is adjusted to about pH 7

| **O/W-Emulsions** | **4.5** | **4.6** | **4.7** |
|---|---|---|---|
| | **wt.-%** | **wt.-%** | **wt.-%** |
| Stearic acid | 2.00 | 3.00 | 3.00 |
| Glycerylstearat | 2.00 | 2.00 | 1.00 |
| Sorbitanstearat | | 1.00 | - |
| Dicaprylyl Ether | 3.00 | 3.00 | - |
| Caprylic acid -/Capric acid triglyceride | 3.00 | 3.00 | - |
| Cetearyl Alcohol | 2.00 | 2.00 | - |
| Cetyl Alcohol | - | - | 1.00 |
| Stearyl Alcohol | - | - | 3.00 |
| Hydrogenated Coco-Glycerides | - | - | 4.00 |
| Mineral Oil | - | - | 3.00 |
| PEG-100 stearat | - | 1.00 | 0.50 |
| Trisodium EDTA | - | - | 1.00 |
| Glycerine | 4.00 | 6.00 | 10.00 |
| Dimethicone | - | - | 1.00 |
| Glycyrrhetic acid | 0.20 | - | - |
| Steviol | 0.20 | 0.50 | 0.10 |
| Erythrulose | - | 4.00 | |
| Phenoxyethanol | 0.40 | 0.40 | 0.40 |
| Parabene | 0.20 | 0.20 | 0.20 |
| Citric acid | - | - | 0.09 |
| Carbomer | 0.20 | 0.20 | 0.20 |
| Perfume | q.s. | q.s. | q.s. |
| Water | ad 100 | ad 100 | ad 100 |

| **O/W-Emulsions** | **4.8** | **4.9** |
|---|---|---|
| | **Wt.-%** | **wt.-%** |
| Glycerylstearate | 3.00 | 4.00 |
| C12-15 Alkylbenzoate | 4.00 | 4.00 |
| Caprylic acid -/Capric acid triglyceride | 3.00 | 2.50 |
| Isopropylstearat | 2.00 | 2.50 |
| Cetyl Alcohol | 2.00 | 2.00 |
| Stearyl Alcohol | 2.00 | 2.00 |
| Glycerin | 3.00 | 5.00 |
| Dimethicone | 0.50 | 2.00 |
| Glycyrrhetic acid | - | 0.10 |
| Steviol | 1.00 | 0.50 |
| Phenoxyethanol | 0.40 | 0.40 |
| Parabene | 0.20 | 0.20 |
| Carbomer | 0.10 | 0.10 |
| Perfume | q.s. | q.s. |
| Water | ad 100 | ad 100 |

The pH of the formulation is adjusted to about pH 7

| **Tanning Spray** | **4.10** |
|---|---|
| | **Wt.-%** |
| Butyl methoxydibenzoylmethane | 5.00 |
| Octocrylene | 10.00 |
| Homosalate | 5.00 |
| Glycerine | 0.50 |
| Steviol | 0.50 |
| Perfume | q.s. |
| Ethanol | ad 100 |

The pH of the formulation is adjusted to about pH 7

| **Emulsions-Fluid** | **4.11** |
|---|---|
| | **Wt.-%** |
| Stearic acid | 2.00 |
| Dicaprylylether | 3.00 |
| Octyldodecanol | 2.00 |
| C12-15 Alkylbenzoate | 4.00 |
| Cetylalcohol | 2.00 |
| Cetearyl Ethylhexanoate + Isopropylmyristate | 2.00 |
| Glycerine | 5.00 |
| Ethylhexylmethoxycinnamate | 2.00 |
| TiO2 | 1.00 |
| Cetylpalmitate | 1.00 |
| Glyceryl Stearate | 1.00 |
| Phenoxyethanol | 0.40 |
| Butyl Methoxydibenzoxylmethane | 2.00 |
| Perfume | q.s. |
| EDTA | 0.20 |
| Carbomer | 0.20 |
| Magnesium Aluminium Silicate | 0.20 |
| Paraben | 0.20 |
| Vitamin E Acetat | 0.10 |
| Steviol | 0.10 |
| Paraben | 0.05 |
| BHT | 0.05 |
| DHA | 1.00 |

| **O/W-Emulsion: night cream** | **4.12** |
|---|---|
| | **Wt.-%** |
| Glycerylstearatcitrate | 2.00 |
| Stearylalcohol | 2.00 |
| Cetylalcohol | 2.00 |
| Hydrogenated Coco Glyceride | 1.00 |
| Caprylic acid -/Capric acid triglyceride | 3.00 |
| Ethylhexylkcoco fatty acid esters | 2.00 |
| Dicaprylylether | 2.00 |
| C12-15 Alkylbenzoate | 3.00 |
| Tocopherylacetate | 1.00 |
| Ubichinon (Coenzym Q10) | 0.10 |
| Sodium ascorbylphosphate | 0.10 |
| Steviol | 1.00 |
| Parabene | 0.40 |
| Methylpropandiol | 1.00 |
| Carrageenan | 0.10 |
| Carbomer | 0.20 |
| Tapioca starch | 2.00 |
| EDTA | 0.20 |
| Glycerine | 5.00 |
| Waster and/ or oil soluble dyes | 0.05 |
| Filling agents/Additives | 0.50 |
| Perfume | q.s. |
| Water | ad 100 |

The pH of the formulation is adjusted to about pH 6.5

| **O/W-Emulsion: day cream** | **4.13** | **4.14** |
|---|---|---|
| | **Wt.-%** | **Wt.-%** |
| PEG-40-Stearat | 1.00 | 1.00 |
| Glycerylstearate | 3.00 | 3.00 |
| Cetearylalcohol | 2.00 | 2.00 |
| Dimethicone | 1.00 | 1.00 |
| Hydrogenated Coco Glycerides | 2.00 | 2.00 |
| Caprylic acid -/Capric acid triglyceride | 2.00 | 2.00 |
| Octyldodecanol e | 2.00 | 2.00 |
| Dicaprylylcarbonate | 2.00 | 2.00 |
| C12-15 Alkylbenzoate | 3.00 | 3.00 |
| Ethylhexyl methoxycinnamate | 4.00 | 4.00 |
| Butyl methoxydibenzoylmethane | 2.00 | 2.00 |
| Tocopherylacetate | 1.00 | 1.00 |
| Panthenol | 0.50 | 0.50 |
| Sodium ascorbylphosphate | 0.10 | 0.10 |
| Ammonium Acryloyldimethyltaurate/VP Copolymer | - | 0.40 |
| Glycyrrhetic acid | - | 0.10 |
| Steviol | 1.00 | 0.50 |
| Nylon-12 | 3.00 | - |
| Distarch phosphate | - | 2.00 |
| Parabene | 0.40 | 0.40 |
| Methylpropandiol | 1.00 | 1.00 |
| Carbomer | 0.20 | 0.20 |
| Xanthan gum | 0.10 | 0.10 |
| EDTA | 0.20 | 0.20 |
| Glycerine | 8.00 | 8.00 |
| Tapioca starch | 0.05 | 0.05 |
| Filling agents/Additives | 0.30 | 0.30 |
| Perfume | q.s. | q.s. |
| Water | ad 100 | ad 100 |

The pH of the formulation is adjusted to about pH 6.5

| **O/W-Emulsion: facial cream** | **4.15** | **4.16** |
|---|---|---|
| | **Wt.-%** | **Wt.-%** |
| Polyglyceryl-3-Methylglucosedistearate | 2.00 | 2.00 |
| Sorbitanstearate | 3.00 | 3.00 |
| Cetylalcohol | 2.00 | 2.00 |
| Myristylmyristate | 1.00 | 1.00 |
| Dicaprylylether | 3.00 | 3.00 |
| Octyldodecanol | 2.00 | 2.00 |
| C12-15 Alkylbenzoate | 3.00 | 3.00 |
| Cetearyl Ethylhexanoat + Isopropylmyristate | 2.00 | 2.00 |
| Ethylhexyl methoxycinnamate | 2.00 | 2.00 |
| Ethylhexyltriazone | 1.00 | 1.00 |
| Butyl Methoxydibenzoxylmethane | 2.00 | 2.00 |
| Magnesium Aluminium Silicate | 0.20 | 0.20 |
| Glycerine | 5.00 | 5.00 |
| Phenoxyethanole | 0.40 | 0.40 |
| Parabene | 0.30 | 0.30 |
| Vitamin E Acetate | 0.10 | 0.10 |
| Glycyrrhetic acid | - | 0.10 |
| Steviol | 1.50 | 1.00 |
| BHT | 0.05 | 0.05 |
| EDTA | 0.20 | 0.20 |
| Carbomer | 2.00 | 0.20 |
| Perfume | q.s. | q.s. |
| Water | ad 100 | ad 100 |

The pH of the formulation is adjusted to about pH 7 (among the compositions of the examples 3 and 4 the following are according to the invention: a) example 3: O/W emulsions 4, 7, 8; hydrodispersions 1, 2, 6; b) example 4: 4.2, 4.6).

## Claims

1. A cosmetic composition for use in a method for increasing the skin tan and/ or preventing or reducing the risk of sun burns said method comprising topically applying onto the skin of an individual seeking such treatment a cosmetic composition comprising at least steviol in an amount ranging from 0.0001-20 wt.-% based on the total weight of the composition formulated into a cosmetically acceptable carrier and at least one coloring agent selected from erythrulose and/ or dihydroxyacetone.

2. The composition according to claim 1, wherein steviol is in the form of a cosmetically acceptable salt thereof.

3. The composition according to claim 1 or 2, wherein the amount of steviol in the composition is selected in the range of 0.01-1 wt.-% based on the total weight of the composition.

4. The composition according to any one of claims 1 to 3, wherein the composition has a pH of 8 or less.

5. The composition according to any one of claims 1 to 4, wherein the pH of the composition is selected in the range of 3 to 7.5.

6. The composition according to any one of claims 1 to 5, wherein the composition further comprises at least one stabilizer and/or at least one photoprotective agent and/or at least one wetting agent and/or at least one penetrant.

7. The composition according to claim 6, wherein the at least one photoprotective agent is selected from the group consisting of octocrylene, 4-methyl benzylidene, ethylhexyl methoxycinnamate, ethylhexyl triazone, diethylhexyl butamido triazone, 2,2'-methylene-bis-(6-(2H-benzotriazole-2-yl)-4-(1,1,3,3,-tetramethylbutyl)-phenol), bis-ethylhexyl-oxyphenol methoxyphenyl triazine, 2,2-(1,4-phenylene)bis-(1H-benzimidazol-4,6-disulfonic acid, 2-(4-Diethylamino-2-hydroxy-benzoyl)-benzoic acid hexylester, 1,1'-(1,4-piperazinediyl)bis[1-[2-[4-(diethylamino)-2-hydroxybenzoyl]- phenyl]-methanone, polysilicone-15, 2-phenyl benzimidazole sulfonic acid, ethylhexyl salicylate, homomenthyl Salicylate, Benzophenone-3, Benzophenone-4, Butyl Methoxydibenzoyl Methane, Terephtalidene dicampher Sulfonic Acid, Drometrizole Trisiloxane and microfine zinc or titanium dioxide as well as mixtures thereof.

## Patentansprüche

1. Kosmetische Zusammensetzung zur Verwendung bei einem Verfahren zur Steigerung der Hautbräune und/oder Prävention oder Reduktion der Gefahr von Sonnenbränden, wobei das Verfahren das topische Aufbringen einer kosmetischen Zusammensetzung, umfassend zumindest Steviol in einer Menge im Bereich von 0,0001 bis 20 Gew.-%, bezogen auf das Gesamtgewicht der Zusammensetzung, die in einen kosmetisch akzeptablen Träger formuliert ist, und mindestens einen Farbstoff, ausgewählt aus Erythrulose und/oder Dihydroxyaceton, auf die Haut eines Individuums, das eine solche Behandlung verlangt.

2. Zusammensetzung nach Anspruch 1, wobei Steviol in der Form eines kosmetisch akzeptablen Salzes davon vorliegt.

3. Zusammensetzung nach Anspruch 1 oder 2, wobei die Steviol-Menge in der Zusammensetzung im Bereich von 0,01 bis 1 Gew.-%, bezogen auf das Gesamtgewicht der Zusammensetzung, ausgewählt ist.

4. Zusammensetzung nach einem der Ansprüche 1 bis 3, wobei die Zusammensetzung einen pH-Wert von 8 oder weniger aufweist.

5. Zusammensetzung nach einem der Ansprüche 1 bis 4, wobei der pH-Wert der Zusammensetzung im Bereich von 3 bis 7,5 ausgewählt ist.

6. Zusammensetzung nach einem der Ansprüche 1 bis 5, wobei die Zusammensetzung zudem mindestens einen Stabilisator und/oder mindestens ein photoprotektives Mittel und/oder mindestens ein Netzmittel und/oder mindestens ein Penetrationsmittel umfasst.

7. Zusammensetzung nach Anspruch 6, wobei das mindestens eine photoprotektive Mittel ausgewählt ist aus der Gruppe, bestehend aus Octocrylen, 4-Methylbenzyliden, Ethylhexylmethoxycinnamat, Ethylhexyltriazon, Diethylhexylbutamidotriazon, 2,2'-Methylen-bis-(6-(2H-benzotriazol-2-yl)-4-(1,1,3,3-tetramethylbutyl)-phenol), Bis-ethylhexyloxyphenolmethoxyphenyltriazin, 2,2-(1,4-Phenylen)bis-(1H-benzimidazol-4,6-disulfonsäure, 2-(4-Diethylamino-2-hydroxybenzoyl)-benzoesäurehexylester, 1,1'-(1,4-Piperazindiyl)bis[1-[2-[4-(diethylamino)-2-hydroxybenzoyl]phenyl]methanon, Polysilikon-15, 2-Phenylbenzimidazolsulfonsäure, Ethylhexylsalicylat, Homomenthylsalicylat, Benzophenon-3, Benzophenon-4, Butylmethoxydibenzoylmethan, Terephthalidendicamphersulfonsäure, Drometrizol-Trisiloxan und mikrofeinem Zink oder Titandioxid sowie Gemischen davon.

## Revendications

1. Composition cosmétique destinée à être utilisée dans une méthode d'augmentation du bronzage cutanée et/ou de prévention ou de réduction du risque de coups de soleil, ladite méthode comprenant l'application de manière topique sur la peau d'un individu recherchant un tel traitement d'une composition cosmétique comprenant au moins du stéviol en une quantité comprise dans la plage allant de 0,0001 à 20 % en poids par rapport au poids total de la composition formulée dans un véhicule cosmétiquement acceptable et au moins un agent colorant choisi parmi l'érythrulose et/ou la dihydroxyacétone.

2. Composition selon la revendication 1, dans laquelle le stéviol est sous la forme d'un sel cosmétiquement acceptable de celui-ci.

3. Composition selon la revendication 1 ou 2, dans laquelle la quantité de stéviol dans la composition est choisie dans la plage allant de 0, 01 à 1 % en poids par rapport au poids total de la composition.

4. Composition selon l'une quelconque des revendications 1 à 3, dans laquelle la composition a un pH inférieur ou égal à 8.

5. Composition selon l'une quelconque des revendications 1 à 4, dans laquelle le pH de la composition est choisi dans la plage de 3 à 7,5.

6. Composition selon l'une quelconque des revendications 1 à 5, dans laquelle la composition comprend en outre au moins un stabilisant et/ou au moins un agent photoprotecteur et/ou au moins un agent mouillant et/ou au moins un agent de pénétration.

7. Composition selon la revendication 6, dans laquelle l'au moins un agent photoprotecteur est choisi dans le groupe constitué par l'octocrylène, le 4-méthylbenzylidène, le méthoxycinnamate d'éthylhexyle, l'éthylhexyltriazone, la diéthylhexylbutamidotriazone, le 2,2'-méthylène-bis-(6-(2H-benzotriazol-2-yl)-4-(1,1,3,3-tétraméthylbutyl)-phénol), la méthoxyphényltriazine de bis-éthylhexyl-oxyphénol, l'acide 2,2-(1,4-phényléne)bis-(1H-benzimidazol-4,6-disulfonique, l'ester hexylique de l'acide 2-(4-diéthylamino-2-hydroxy-benzoyl)-benzoique, la 1,1'-(1,4-pipérazinediyl)bis[1-[2-[4-(diéthylamino)-2-hydroxybenzoyl]-phényl]-méthanone, la polysilicone-15, l'acide 2-phénylbenzimidazole-sulfonique, le salicylate d'éthylhexyle, le salicylate d'homomenthyle, la benzophénone-3, la benzophénone-4, le butylméthoxydibenzoylméthane, l'acide téréphtalidène-dicamphre-sulfonique, le drométrizole-trisiloxane et le zinc microfin ou le dioxyde de titane microfin ainsi que des mélanges de ceux-ci.
